Europäisches Patentamt
European Patent Office
Office européen des brevets

(19) (11) Publication number: **0 269 238 A1**

(12) **EUROPEAN PATENT APPLICATION**

(21) Application number: 87309141.7

(22) Date of filing: 15.10.87

(51) Int. Cl.4: **A01N 43/50**, C07D 233/92, C07D 233/90

The title of the invention has been amended (Guidelines for Examination in the EPO, A-III, 7.3).

(30) Priority: 17.10.86 GB 8624879

(43) Date of publication of application: 01.06.88 Bulletin 88/22

(84) Designated Contracting States: AT BE CH DE ES FR GB GR IT LI LU NL SE

(71) Applicant: MAY & BAKER LIMITED
Dagenham Essex RM10 7XS(GB)

(72) Inventor: Feldwick, Graham Alan
c/o May & Baker Limited
Dagenham Essex, RM10 7XS(GB)
Inventor: Hatton, Leslie Roy
c/o May & Baker Limited
Dagenham Essex, RM10 7XS(GB)
Inventor: Hewett, Richard Henry
c/o May & Baker Limited
Dagenham Essex, RM10 7XS(GB)
Inventor: Pedgrift, Brian Leslie
c/o May & Baker Limited
Dagenham Essex, RM10 7XS(GB)

(74) Representative: Bentham, Stephen et al
J.A. Kemp & Co. 14 South Square Gray's Inn
London WC1R 5EU(GB)

(54) **Imidazoles and their compositions for plant growth regulation.**

(57) The invention provides a method of regulating the growth of a plant using a compound of the formula:

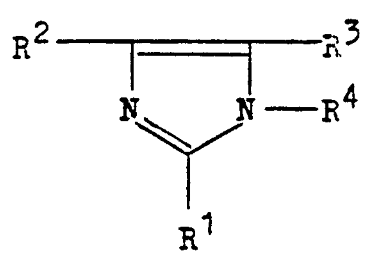

I

wherein $R^1$ represents alkyl or phenyl optionally substituted by halogen, alkyl, alkoxy, alkylthio, trifluoromethyl, trifluoromethoxy, trifluoromethylthio, nitro, cyano, amino, carboxy, phenoxy, benzyloxy, alkoxycarbonyl, alkylamino, dialkylamino, alkanoylamino, $R^2$ represents nitro, cyano, carbamoyl or $-CONR^5R^6$ or $-NR^6COR^5$ (wherein $R^5$ is as hereinbefore defined for $R^1$ and $R^6$ represents hydrogen or alkyl), $R^3$ represents halogen, nitro, cyano, amino, carbamoyl, $-CONR^7R^8$ (wherein $R^7$ is as hereinbefore defined for $R^1$ and $R^8$ represents hydrogen or alkyl), and $R^4$ represents hydrogen or alkyl, or salts thereof with the exclusion of compounds in which $R^2$ and $R^3$ simultaneously represent cyano groups; plant-growth regulating compositions, novel compounds and their preparation are described.

EP 0 269 238 A1

## "IMIDAZOLES"

The present invention relates to a method for regulating plant growth by applying imidazole derivatives to the plants, to plant material and/or their environment and to compositions for regulating plant growth containing at least one imidazole derivative as an active ingredient, and to novel imidazole derivatives and a process for their preparation.

Many organic chemicals affect the growth of plants. More often than not, application of an organic chemical to plants has a detrimental effect, including death of the plants. Such chemicals are referred to as herbicides. Occasionally compounds are discovered which have beneficial effects on plants, but are not simply nutrient-supplying fertilizers. Such beneficial effects include increasing the yield of fruit, seeds, fibre, or other plant products. Another beneficial effect may be an increase in the nutritional value of food products derived from the plants. It is a beneficial effect of some compounds to facilitate harvesting the plant product. Yet another beneficial effect in certain cases is an increase in the storage life of the plant product. Such non-nutrient chemical compounds which are beneficial to plants in small amounts are referred to as plant growth regulators.

As used herein, the term "plant growth regulator" means a chemical compound which, when applied in the recommended manner, modifies the normal growth of a plant or part of a plant. Such modifications may include, but are not limited to: root initiation; set, development, ripening and abscission of fruits; plant size and shape; suppression of lodging; control of axillary buds and lateral shoots; metabolism regulation, including senescence; breaking or enforcing dormancy in seeds, buds, and storage organs; promotion or delay of flowering; defoliation; desiccation; and growth promotion under stress.

They can be used to sustain and regulate division and growth of plant cells, cell aggregates, embryonic tissue and explants in cell tissue culture and micropropagation. They can also be applied as seed dressings to promote germination and early growth of plants. They can be applied exogenously to the aerial parts of plants to substantially improve yield of vegetative and reproductive parts.

Applications are particularly beneficial to plants used for ornamental, amenity, industrial and food purposes.

Although a plant growth regulator, as that term is used herein, may inhibit plant growth even when the compound is properly used, the term does not contemplate total growth inhibition or killing the plant when the regulator is used as recommended.

A number of imidazole compounds are known from the literature. However, although compounds falling within the scope of the general formula shown in Figure I at the end of the present specification as defined hereinafter are known there has been no disclosure of the use of such compounds as plant growth regulators nor any suggestion that it might be desirable to test the compounds as plant growth regulators.

(a) Y. Ohtsuka, in J.Org.Chem., Vol. 44, No. 5, 1979, p.p. 827-830 discloses the compound of the general formula shown in Figure I wherein $R^1$ represents an unsubstituted phenyl group, $R^2$ represents a cyano group, $R^3$ represents a carbamoyl group and $R^4$ represents a hydrogen atom, but discloses no utility for the compound and contains no suggestion of any biological activity;

(b) the specification of United States Patent No. 3836658 discloses the compounds of the general formula shown in Figure I wherein $R^1$ represents a tert-butyl or isopropyl group, or a phenyl group carrying one substituent selected from halogen atoms and alkoxy and alkanoylamino groups (substitution by one methyl group is also disclosed), $R^2$ and $R^2$ may be the same or different and each represents a cyano or carbamoyl group or a group of the formula $-CONR^5R^6$ or $-CONR^7R^8$ (wherein $R^5$ or $R^7$ represents an alkyl group and $R^6$ or $R^8$ represents a hydrogen atom), and $R^4$ represents a hydrogen atom, and salts thereof, and teaches that they have pharmaceutical utility as antihyperuricemic agents in the treatment of gout;

(c) the specification of Belgian Patent No. 741077 discloses the compounds of the general formula shown in Figure I wherein $R^1$ represents an alkyl group containing from 1 to 4 carbon atoms, $R^2$ represents a nitro or cyano group, $R^3$ represents a halogen atom or a nitro, cyano or amino group and $R^4$ represents a hydrogen atom or an alkyl group containing from 1 to 4 carbon atoms, and teaches that they have pharmaceutical antitrichomonal and antiviral activity;

(d) the specification of United States Patent No. 3501286 discloses the compounds of the general formula shown in Figure I wherein $R^1$ represents an alkyl group containing from 1 to 4 carbon atoms, $R^2$ represents a nitro or cyano group, $R^3$ represents a halogen atom or a nitro or cyano group and $R^4$ represents a hydrogen atom or an alkyl group, and teaches that they are useful as herbicides, with no suggestion that they may have any effect on a plant which is beneficial to that plant, i.e. with no suggestion of utility as plant growth regulators as hereinbefore defined;

(e) the specification of United States Patent No. 3806517 mentions that compounds of the general formula shown in Figure I wherein $R^1$ represents an alkyl group, $R^2$ and $R^3$ each represent carbamoyl groups and $R^4$ represents a hydrogen atom may be prepared by hydrolysis of corresponding 4,5-dicyano compounds and states that they in turn may be converted into xanthines of use in medicine or in beverages;

(f) the specification of United States Patent No. 3968228 discloses the compounds of the general formula shown in Figure I wherein $R^1$ represents a phenyl group substituted with halogen atoms or nitro groups, $R^2$ represents a nitro group, $R^3$ represents a cyano group and $R^4$ represents an alkyl group containing from 1 to 5 carbon atoms, and teaches their utility in the prevention and treatment of coccidiosis in poultry;

(g) the specification of United States Patent No. 3959305 discloses the compounds of the general formula shown in Figure I wherein $R^1$ represents an alkyl group, $R^2$ represents a group of the formula $-NR^6COR^5$ (wherein $R^5$ represents an alkyl group containing from 1 to 7 carbon atoms and $R^6$ represents a hydrogen atom), $R^3$ represents a carbamoyl group or a group of the formula $-CONR^7R^8$ (wherein $R^7$ represents an alkyl group containing from 1 to 4 carbon atoms and $R^8$ represents a hydrogen atom or an alkyl group containing from 1 to 4 carbon atoms) and $R^4$ represents a hydrogen atom or an alkyl group, and teaches their pharmaceutical anti-inflammatory activity, and also discloses compounds of the general formula shown in Figure I wherein $R^1$ represents an alkyl group, $R^2$ represents a carbamoyl group or a group of the formula $-CONR^5R^6$ (wherein $R^5$ represents an alkyl group containing from 1 to 4 carbon atoms and $R^6$ represents a hydrogen atom or an alkyl group containing from 1 to 4 carbon atoms), $R^3$ represents an amino group and $R^4$ represents a hydrogen atom or an alkyl group, which are intermediates for the preparation of some of the said anti-inflammatory compounds;

(h) the specification of United States Patent No. 3772319 discloses in its broadest disclosure the compounds of the general formula shown in Figure I wherein $R^1$ represents a phenyl group optionally substituted on its 3-, 4-, 5-and 6-positions by one or two substituents selected from halogen atoms, alkyl groups containing from 1 to 4 carbon atoms and trifluoromethyl, nitro, amino, carboxy, etherified hydroxy, alkoxycarbonyl, ålkylamino, dialkylamino and alkanoylamino groups, $R^2$ represents an amide group, $R^3$ represents an amino group and $R^4$ represents a hydrogen atom, and salts thereof, and teaches their pharmaceutical anti-inflammatory activity;

(i) the specification of United States Patent No. 3876655 discloses the compounds of the general formula shown in Figure I wherein $R^1$ represents a phenyl group optionally substituted by a halogen atom or a methyl, methoxy, trifluoromethyl or nitro group, $R^2$ represents a group of the formula $-NR^6COR^5$ (wherein $R^5$ represents an alkyl group containing 1 to 7 carbon atoms and $R^6$ represents a hydrogen atom), $R^3$ represents a carbamoyl group and $R^4$ represents a hydrogen atom, and teaches their pharmaceutical anti-inflammatory activity;

(j) the specification of Belgian Patent No. 777738 discloses the compounds of the general formula shown in Figure I wherein $R^1$ represents a phenyl group optionally substituted on its 3-, 4-, 5-and 6-positions by one or two substituents selected from halogen atoms, alkyl groups containing from 1 to 4 carbon atoms and trifluoromethyl, nitro, amino, carboxy, etherified hydroxy, alkoxycarbonyl, alkylamino, dialkylamino and alkanoylamino groups, $R^2$ represents a group of the formula $-NR^6COR^5$ (wherein $R^5$ represents an alkyl group containing from 1 to 7 carbon atoms and $R^6$ represents a hydrogen atom or an alkyl group), $R^3$ represents a carboxamide group, and $R^4$ represents a hydrogen atom, and salts thereof, and teaches their pharmaceutical anti-inflammatory activity; and

(k) the specification of British Patent No. 1399291 discloses the compounds of the formula shown in Figure I wherein $R^1$ represents an alkyl group containing from 1 to 5 carbon atoms or a phenyl group substituted by one or more substituents selected from halogen atoms and nitro groups, $R^2$ represents a nitro group, $R^3$ represents a cyano group, and $R^4$ represents an alkyl group containing from 1 to 5 carbon atoms, and teaches their activity in the prevention and treatment of coccidiosis in poultry [c.f. paragraph (f) above].

According to a feature of the present invention there is provided a method of regulating the growth of a plant or of plant material at a locus which comprises applying to the plant, to plant material or to the locus an amount, effective to regulate growth, of a compound of the general formula shown in Figure I at the end of the present specification, wherein $R^1$ represents a straight-or branched-chain alkyl group containing from 1 to 8 carbon atoms or a phenyl group which is optionally substituted by one or more substituents selected from halogen (i.e. chlorine, fluorine, bromine and iodine) atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, alkoxycarbonyl, alkylamino and dialkylamino groups in which the alkyl moieties are each straight or branched and contain from 1 to 6 carbon atoms, and

straight-or branched-chain alkanoylamino groups containing from 2 to 5 carbon atoms, $R^2$ represents a nitro, cyano or carbamoyl group or a group of the formula $-CONR^5R^6$ or $-NR^6COR^5$ (wherein $R^5$ is as hereinbefore defined for $R^1$, and $R^6$ represents a hydrogen atom or a straight-or branched-chain alkyl group containing from 1 to 6 carbon atoms), $R^3$ represents a halogen (i.e. chlorine, bromine, iodine or fluorine) atom or a nitro, cyano, amino or carbamoyl group or a group of the formula $CONR^7R^8$ (wherein $R^7$ is as hereinbefore defined for $R^1$, and represents a hydrogen atom or a straight-or branched-chain alkyl group containing from 1 to 6 carbon atoms), and $R^4$ represents a hydrogen atom or a straight or branched-chain alkyl group containing from 1 to 6 carbon atoms, or an agriculturally acceptable salt thereof, with the exclusion of compounds in which $R^2$ and $R^3$ simultaneously represent cyano groups.

A preferred method of the invention for increasing the yield of a root, seed or fruit crop at a locus comprises applying thereto an effective amount of a compound of general formula I.

A further preferred method of the invention comprises dressing seed, prior to planting, with a composition comprising a compound of general formula I.

The invention also provides a method of increasing the growth of a plant cell culture by growing the cell culture in a medium comprising a compound of general formula I.

The person skilled in the art will perceive that certain of the compounds of the formula shown in Figure I, for example those containing amino groups, will tend to have basic properties whilst certain of the compounds, for example those containing carboxy groups, will tend to have acidic properties. Agriculturally acceptable salts formed by such compounds with acids or bases, respectively, are included in the scope of the formula shown in Figure I and it is to be understood that references to compounds of the formula shown in Figure I also embrace the salts whenever the context so permits.

By the term "agriculturally acceptable salts" as used in this specification is meant salts of acids or of bases which are known and accepted in the art for the formation of salts of biologically active compounds for agricultural or horticultural use. Suitable salts of acids include salts of inorganic acids such as hydrochlorides, sulphates, phosphates and nitrates and salts of organic acids, for example acetates. Suitable salts of bases include alkali metal, e.g. sodium or potassium, salts, alkaline earth metal, e.g. calcium or magnesium, salts and salts of ammonia or of organic bases, e.g. amines (e.g. diethanolamine or triethanolamine).

The person skilled in the art will perceive that when $R^4$ represents a hydrogen atom tautomerism occurs whereby the nitrogen atoms at positions 1 and 3 in the imidazole ring become equivalent to each other and consequently the carbon atoms at positions 4 and 5 in the imidazole ring become equivalent to each other. In these circumstances the substituents $R^2$ can be within the definition of $R^3$ and, simultaneously, $R^3$ can be within the definition of $R^2$.

Thus, as a feature of the invention there are provided the compounds of the general formula shown in Figure I wherein $R^1$ represents a straight-or branched-chain alkyl group containing from 1 to 8 carbon atoms or a phenyl group which is optionally substituted by one or more substituents selected from halogen (i.e. chlorine, fluorine, bromine and iodine) atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, alkoxycarbonyl, alkylamino and dialkylamino groups in which the alkyl moieties are each straight or branched and contain from 1 to 6 carbon atoms, and straight- or branched-chain alkanoylamino groups containing from 2 to 5 carbon atoms, $R^2$ represents a nitro, cyano or carbamoyl group or a group of the formula $-CONR^5R^6$ or $-NR^6COR^5$ (wherein $R^5$ is as hereinbefore defined for $R^1$, and $R^6$ represents a hydrogen atom or a straight-or branched-chain alkyl group containing from 1 to 6 carbon atoms), $R^3$ represents a halogen (i.e. chlorine, bromine, iodine or fluorine) atom or a nitro, cyano, amino or carbamoyl group or a group of the formula $-CONR^7R^8$ (wherein $R^7$ is as hereinbefore defined for $R^1$, and $R^8$ represents a hydrogen atom or a straight-or branched-chain alkyl group containing from 1 to 6 carbon atoms), and $R^4$ represents a hydrogen atom or a straight-or branched-chain alkyl group containing from 1 to 6 carbon atoms, or an agriculturally acceptable salt thereof, with the exclusion of compounds in which $R^2$ and $R^3$ simultaneously represent cyano groups, and with the exclusion of:-

compounds of the general formula shown in Figure I wherein $R^1$ represents an unsubstituted phenyl group, $R^2$ represents a cyano group, $R^3$ represents a carbamoyl group and $R^4$ represents a hydrogen atom;

compounds of the general formula shown in Figure I wherein $R^1$ represents a <u>tert</u> -butyl or isopropyl group, or a 4-methylphenyl, 4-chlorophenyl, 4-acetamidophenyl or 4-methoxyphenyl group, or more generally a phenyl group carrying one substituent selected from halogen atoms and alkyl, alkoxy and alkanoylamino groups, $R^2$ and $R^3$ may be the same or different and each represents a cyano or carbamoyl group or a group of the formula $-CONR^5R^6$ or $-CONR^7R^8$ (wherein $R^5$ or $R^7$ represents an alkyl group and $R^6$ or $R^8$ represents a hydrogen atom), and $R^4$ represents a hydrogen atom, and salts thereof;

compounds of the general formula shown in Figure I wherein $R^1$ represents an alkyl group containing

from 1 to 4 carbon atoms, $R^2$ represents a nitro or cyano group, $R^3$ represents a halogen atom or a nitro, cyano or amino group and $R^4$ represents a hydrogen atom or an alkyl group containing from 1 to 4 carbon atoms;

compounds of the general formula shown in Figure I wherein $R^1$ represents an alkyl group containing from 1 to 4 carbon atoms, $R^2$ represents a nitro or cyano group, $R^3$ represents a halogen atom or a nitro or cyano group and $R^4$ represents a hydrogen atom or an alkyl group;

compounds of the general formula shown in Figure I wherein $R^1$ represents an alkyl group, $R^2$ and $R^3$ each represent carbamoyl groups and $R^4$ represents a hydrogen atom;

compounds of the general formula shown in Figure I wherein $R^1$ represents an alkyl group, $R^2$ represents a group of the formula $-NR^6COR^5$ (wherein $R^5$ represents an alkyl group containing from 1 to 7 carbon atoms and $R^6$ represents a hydrogen atom), $R^3$ represents a carbamoyl group or a group of the formula $-CONR^7R^8$ (wherein $R^7$ represents an alkyl group containing from 1 to 4 carbon atoms and $R^8$ represents a hydrogen atom or an alkyl group containing from 1 to 4 carbon atoms) and $R^4$ represents a hydrogen atom or an alkyl group;

compounds of the general formula shown in Figure I wherein $R^1$ represents an alkyl group, $R^2$ represents a carbamoyl group or a group of the formula $-CONR^5R^6$ (wherein $R^5$ represents an alkyl group containing from 1 to 4 carbon atoms and $R^6$ represents a hydrogen atom or an alkyl group containing from 1 to 4 carbon atoms), $R^3$ represents an amino group and $R^4$ represents a hydrogen atom or an alkyl group;

compounds of the general formula shown in Figure I wherein $R^1$ represents a phenyl group optionally substituted on its 3-, 4-, 5-and 6-positions by one or two substituents selected from halogen atoms, alkyl groups containing from 1 to 4 carbon atoms and alkoxy, trifluoromethyl, nitro, amino, carboxy, phenoxy, benzyloxy, alkoxycarbonyl, alkylamino, dialkylamino and alkanoylamino groups, $R^2$ represents a group of the formula $-CONR^5R^6$ [wherein $R^5$ represents a straight-or branched-chain alkyl group containing from 1 to 8 carbon atoms or a phenyl group which is optionally substituted by one or more substituents selected from halogen (i.e. chlorine, fluorine, bromine and iodine) atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benxyloxy groups, alkoxycarbonyl, alkylamino and dialkylamino groups in which the alkyl moieties are each straight or branched and contain from 1 to 6 carbon atoms, and straight-or branched-chain alkanoylamino groups containing from 2 to 5 carbon atoms, and $R^6$ represents a hydrogen atom or a straight-or branched-chain alkyl group containing from 1 to 6 carbon atoms], $R^3$ represents an amino group and $R^4$ represents a hydrogen atom, and salts thereof;

compounds of the general formula shown in Figure I wherein $R^1$ represents a phenyl group optionally substituted by a halogen atom or a methyl, methoxy, trifluoromethyl or nitro group, $R^2$ represents a group of the formula $-NR^6COR^5$ (wherein $R^5$ represents an alkyl group containing 1 to 7 carbon atoms and $R^6$ represents a hydrogen atom), $R^3$ represents a carbamoyl group and $R^4$ represents a hydrogen atom;

compounds of the general formula shown in Figure I Wherein $R^1$ represents a phenyl group optionally substituted on its 3-, 4-, 5-and 6-positions by one or two substituents selected from halogen atoms, alkyl groups containing from 1 to 4 carbon atoms and alkoxy, trifluoromethyl, nitro, amino, carboxy, phenoxy, benzyloxy, alkoxycarbonyl, alkylamino, dialkylamino and alkanoylamino groups, $R^2$ represents a group of the formula $-NR^6COR^5$ (wherein $R^5$ represents an alkyl group containing from 1 to 7 carbon atoms and $R^6$ represents a hydrogen atom or an alkyl group), $R^3$ represents a group of the formula $-CONR^7R^8$ [wherein $R^7$ represents a straight-or branched-chain alkyl group containing from 1 to 8 carbon atoms or a phenyl group which is optionally substituted by one or more substituents selected from halogen (i.e. chlorine, fluorine, bromine and iodine) atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, alkoxycarbonyl, alkylamino and dialkylamino groups in which the alkyl moieties are each straight or branched and contain from 1 to 6 carbon atoms, and straight-or branched-chain alkanoylamino groups containing from 2 to 5 carbon atoms, and $R^8$ represents a hydrogen atom or a straight-or branched-chain alkyl group containing from 1 to 6 carbon atoms], and $R^4$ represents a hydrogen atom, and salts thereof; and

compounds of the formula shown in Figure I wherein $R^1$ represents an alkyl group containing from 1 to 5 carbon atoms or a phenyl group substituted by one or more substituents selected from halogen atoms and nitro groups, $R^2$ represents a nitro group, $R^3$ represents a cyano group, and $R^4$ represents an alkyl group containing from 1 to 5 carbon atoms.

Preferred classes of novel compounds of the formula shown in Figure I include those wherein one or more of the symbols $R^1$, $R^2$, $R^3$ and $R^4$ is as defined below, the other symbols $R^1$, $R^2$, $R^3$ and $R^4$ being as hereinbefore defined:-

(i) $R^1$ represents a phenyl group optionally bearing one or two substituents selected from halogen atoms (especially chlorine, bromine or fluorine atoms), trifluoromethyl, trifluoromethoxy, nitro, amino, carboxy, phenoxy and benzyloxy groups, and alkyl, alkoxy and dialkylamino groups in which the alkyl moieties are straight-or branched-chain and each contains from 1 to 6 carbon atoms, and straight-or branched-chain alkanoylamino groups containing from 2 to 5 carbon atoms, e.g. methyl, methoxy, dimethylamino, diethylamino and acetamido groups, especially when $R^1$ represents a phenyl group bearing such substituent(s) in the meta-or, more particularly, the para-position;

(ii) $R^2$ represents a carbamoyl or cyano group;

(iii) $R^3$ represents a carbamoyl or cyano group;

and

(iv) $R^4$ represents a hydrogen atom, and their agriculturally acceptable salts.

Compounds wherein one of $R^2$ and $R^3$ represents a carbamoyl group and the other represents a cyano group, are especially important.

Especially preferred novel compounds of general formula I are those of the general formula shown in Figure IA wherein $R^{1A}$represents a phenyl group substituted by one or two substituents selected from those listed in the definition of $R^1$ in the general formula shown in Figure I, particularly those in which $R^{1A}$ is other than 4-methylphenyl, 4-chlorophenyl, 4-acetamidophenyl or 4-methoxyphenyl.

$R^{1A}$ preferably represents a monosubstituted phenyl group in which the substituent is other than alkyl, alkoxy, alkanoylamino or halogen, or a disubstituted phenyl group.

Compounds of the formula shown in Figure I which are particularly important and which have not hitherto been specifically disclosed include the following and they, the process for their preparation, their compositions and the method of their use as plant growth regulators form features of the present invention.

2-(4-chlorophenyl)-5-cyanoimidazole-4carboxamide A
2-(3-methoxyphenyl)-5-cyanoimidazole-4-carboxamide B
2-(3-nitrophenyl)-5-cyanoimidazole-4-carboxamide C
2-(4-methoxyphenyl)-5-cyanoimidazole-4-carboxamide D
2-(4-nitrophenyl)-5-cyanoimidazole-4-carboxamide E
2-(4-fluorophenyl)-5-cyanoimidazole-4-carboxamide F
2-(4-bromophenyl)-5-cyanoimidazole-4-carboxamide G
2-(4-dimethylaminophenyl)-5-cyanoimidazole-4-carboxamide H
2-(4-diethylaminophenyl)-5-cyanoimidazole-4-carboxamide I
2-(4-acetamidophenyl)-5-cyanoimidazole-4-carboxamide J
2-(4-carboxyphenyl)-5-cyanoimidazole-4-carboxamide K
2-(3,4-dichlorophenyl)-5-cyanoimidazole-4-carboxamide L
2-(4-methylphenyl)-5-cyanoimidazole-4-carboxamide M
2-(2-methylphenyl)-5-cyanoimidazole-4-carboxamide N
2(2-trifluoromethylphenyl)-5-cyanoimidazole-4-carboxamide O
2-(2-chlorophenyl)-5-cyanoimidazole-4-carboxamide P
2-(4-trifluoromethylphenyl)-5-cyanoimidazole-4-carboxamide Q
2-(4-phenoxyphenyl)-5-cyanoimidazole-4-carboxamide R
2-(3-bromophenyl)-5-cyanoimidazole-4-carboxamide S
2-(3-fluorophenyl)-5-cyanoimidazole-4-carboxamide T
2-(3-chlorophenyl)-5-cyanoimidazole-4-carboxamide U
2-(2-fluorophenyl)-5-cyanoimidazole-4-carboxamide V
2-(2,4-dichlorophenyl)-5-cyanoimidazole-4-carboxamide W
2-(4-benzyloxyphenyl)-5-cyanoimidazole-4-carboxamide X
2-(4-trifluoromethoxyphenyl)-5cyanoimidazole-4-carboxamide and Y
2-(4-aminophenyl)-5-cyanoimidazole-4-carboxamide Z

and their agriculturally acceptable salts.

Compounds B, F, G, I, L, S, U, and Z and, more especially, A, are of outstanding importance.

The letters A to Z are assigned to the compounds for easy reference.

The activity of the compounds of the formula shown in Figure I is illustrated by the following tests:-

(i) Foliar application to radishes (Rhaphanus sativus)

Radish seeds (variety "French Breakfast") were sown into pots (9 cm) containing peat-based potting compost and after emergence they were thinned out so that two remained in each pot. When the plants had two pairs of leaves each, groups of 10 pots were sprayed with solutions of various concentrations of compound A in aqueous acetone (ratio of acetone to water 4:1 v/v), at a rate of 500 l/ha.

After 16 days in the greenhouse the weights of the leaves and the fresh and dried weights of the roots were determined, calculating the mean weight per pot, and compared with untreated controls. The results were as shown in Table I below.

TABLE I

| Dose of compound A (kg/ha) | Fresh weight of leaves (g) | Fresh weight of roots (g) | Dried weight of roots (g) |
|---|---|---|---|
| 0 | 9.9 | 5.0 | 0.26 |
| 0.125 | 11.9 | 7.6 | 0.39 |
| 0.25 | 13.6 | 10.1 | 0.52 |

(ii) Application to French dwarf bean

Seeds of French dwarf bean variety 'Rondina' were sown in seed trays of 'Levington 'peat-based potting compost. On emergence young plants were selected for evenness and pricked out one per 9 cm square pot in 'John Innes' loam based potting compost. Plants were grown under glasshouse conditions with supplementary lighting as necessary to provide a 14 hour day length.

As the first trifoliate leaf was starting to expand, plants were sprayed with acetone or aqueous acetone solutions of test compounds at rates of 8, 32, 125 and 500 g/ha using a laboratory sprayer at 500 l/ha. One or more compounds were tested at a time and compound A was included in each test for comparison. Four or more replicate plants were used for each dose rate. Plants were returned to the glasshouse after application of test compound.

After 14 days the first trifoliate leaf of each plant was detached from its petiole and weighed. A 10 $cm^2$ disc was then punched from the left-hand leaflet, weighed, and cut into 6 to 8 pieces and placed into a plastic vial and treated with acetone (10 ml).

After 5 to 7 days the chlorophyll in the leaf pieces had been extracted into solution and its concentration was read in a spectrophotometer using the 645 nm and 663 nm wavelengths.

Total chlorophyll content per disc was calculated using the formula:-

$$\frac{(\text{Optical Density at } 645\text{ nm} \times 20.2) + (\text{Optical Density at } 663\text{ nm} \times 8.02)}{100}$$

Chlorophyll content per gram of leaf and total chlorophyll per leaf were also calculated and the replicate results were averaged.

Comparisons of activity in increasing total chlorophyll per leaf were made between each test compound and compound A, to produce one of the following scores:-

| Score | Meaning |
|---|---|
| 0 | No activity |
| 1 | Trace activity |
| 2 | Ratio of activity of compound A to activity of test compound = 32:1 to 64:1 |
| 3 | Ratio of activity of compound A to activity of test compound = 8:1 to 16:1 |
| 4 | Ratio of activity of compound A to activity of test compound = 2:1 to 4:1 |
| 5 | Activity equal to that of compound A |
| 6 | Ratio of activity of test compound to activity of compound A = 2:1 to 4:1 |

The scores obtained are summarised in the following Table II:-

TABLE II

| Test compound | A | B | C | D | E | F | G |
|---|---|---|---|---|---|---|---|
| Score | 5 | 4 | 1 | 3 | 1 | 5 to 6 | 4 to 5 |

Cont/d.....

TABLE II (Continued)

| Test compound | H | I | J | K | L | M | N | Q | R |
|---|---|---|---|---|---|---|---|---|---|
| Score | 1 | 4 | 2 | 2 | 6 | 3 | 1 | 3 | 1 |

| Test compound | S | T | U | V | W | Z |
|---|---|---|---|---|---|---|
| Score | 4 | 3 | 4 to 6 | 3 | 1 | 4 |

(iii) Foliar application to sugar beet (Beta vulgaris)

Sugar beet seeds (variety 'Bush Mono M35') were sown into a tray of 'Levington' peat-based potting compost and then pricked out at the cotyledon stage into pots (12.7 cm size), three per pot, containing 'John Innes' loam-based potting compost (No. 1). Plants were thinned to one per pot at 1-or 2-leaf stage.

At the 5-or 6-leaf stage the plants were sprayed with solutions of various concentrations of compound A in aqueous acetone (ratio of acetone to water 4:1 v/v) to give dose rates of 0, 63, 125 and 250 g/ha at a volume rate of 500 l/ha. There were 10 pots per concentration.

After 46 days in the glasshouse the weights of the leaves and roots per pot were determined and averaged. The results are given below in Table III.

TABLE III

| Dose rate (g/ha) | Fresh weight of leaves (g) | Fresh weight of root (g) |
|---|---|---|
| 63 | 111 | 37.5 |
| 125 | 117 | 34.4 |
| 250 | 117 | 31.4 |
| Control | 90 | 25.3 |

(iv) Application to turnip under field conditions

Turnip (variety 'Snowball') was drilled in May using standard farm equipment.

NPK fertiliser (13:13:20) was applied before emergence at 700 kg/ha and herbicide application was made as necessary.

At the 5-or 6-leaf stage compound A was applied, using dilutions of the 25% w/v flowable formulation prepared as described hereinafter in Composition Example 7, by means of a motorised small plot crop sprayer at a volume rate of 255 l/ha. Application was made at 0, 0.063, 0.125, 0.250 and 0.500 kg/ha to four replicate plots per concentration, each plot measuring 12 rows by 2.5 m.

At maturity all plants within the central portion (1.5 m) of the central 8 rows were lifted. Leaves were discarded and the roots were cleaned and weighed. The results are shown below in Table IV.

TABLE IV

| dose (kg/ha) | Yield (kg) | Weight per root (g) |
|---|---|---|
| 0.063 | 16.5 | 148 |
| 0.125 | 17.1 | 160 |
| 0.250 | 17.5 | 161 |
| 0.500 | 17.3 | 148 |
| Control | 15.9 | 136 |

(v) Application to maize (Zea mays variety 'Fronica') as a seed dressing

Maize seeds were dressed by dipping them in aqueous acacia gum solution (5% w/v) and by rolling in a finely divided mixture of silica and compound A. The quantity of compound A adhering to each seed was determined by weighing.

Pots (10 cm) of loam-based potting compost were each sown with four dressed seeds and were kept in the greenhouse under sodium lamps, watering once daily. The seedlings were thinned out so that there were three in each pot, and three such pots were used for each treatment.

The lengths of the various leaves (the distance between the soil surface and the end of the leaf) were measured at intervals and compared with the results from untreated seeds.

The results obtained from treating the seeds with 0.86 mg per seed were as shown hereinbelow in Table V, which shows the percentage increase in length found for each leaf, compared with the controls.

TABLE V

| | Time (days) after sowing | | | | |
|---|---|---|---|---|---|
| | 7 | 12 | 14 | 19 | 28 |
| Leaf 1 | -3 | -4 | | | |
| Leaf 2 | +21 | 0 | -1 | | |
| Leaf 3 | | +10 | +2 | -3 | |
| Leaf 4 | | +27 | +15 | +3 | -1 |
| Leaf 5 | | | +7 | +15 | +1 |
| Leaf 6 | | | | +13 | +2 |
| Leaf 7 | | | | | +13 |
| Leaf 8 | | | | | +22 |

(vi) Foliar application to winter wheat (triticum aestivum)

Pots (12.7 cm size) containing 'John Innes' loam-based potting compost No. 1 were sown with six seeds each of winter wheat (variety 'Avalon') in October and placed outside on gravel under bird-proof netting. Plants were thinned to three per pot in March. At the first node stage groups of 15 pots were sprayed with solutions of various concentrations of compound A in aqueous acetone (ratio of acetone to water 4:1 v/v) to give dose rates of 0, 0.25, 0.50 and 1.00 kg/ha at a volume rate of 500 l/ha.

On maturity of the plants the heads were removed and the head number was determined. The heads were threshed using a laboratory thresher and the collected seed was placed in an oven at 60°C for three days. Seed was then weighed (dry weight) and counted. 1000 grain weight, dry weight per head and seed number per head were calculated and the mean of each parameter was determined. The results are shown below in Table VI.

TABLE VI

| Dose (kg/ha) | Number of heads | Dry weight of seeds | Number of seeds |
|---|---|---|---|
| 0.25 | 7.3 | 12.7 | 333 |
| 0.50 | 7.4 | 11.8 | 327 |
| 1.00 | 6.8 | 10.2 | 300 |
| Control | 6.3 | 9.4 | 281 |

| Dose (kg/ha) | Dry weight per head | Seeds per head | 1000 grain dry wt (g) |
|---|---|---|---|
| 0.25 | 1.8 | 46 | 38.4 |
| 0.50 | 1.6 | 45 | 35.2 |
| 1.00 | 1.5 | 44 | 34.2 |
| Control | 1.6 | 46 | 33.9 |

(vii) Foliar application to winter barley (Hordeum vulgare)

The procedure for winter wheat was repeated using winter barley (variety 'Igri') and the results are given below in Table VII.

TABLE VII

| Dose (kg/ha) | Number of heads | Dry weight of seeds | Number of seeds |
|---|---|---|---|
| 0.25 | 22.6 | 12.5 | 361 |
| 0.50 | 24.5 | 12.9 | 357 |
| 1.00 | 19.1 | 6.1 | 224 |
| Control | 20.6 | 9.1 | 303 |

| Dose (kg/ha) | Dry weight per head | Seeds per head | 1000 grain dry wt (g) |
|---|---|---|---|
| 0.25 | 0.6 | 17 | 33.3 |
| 0.50 | 0.5 | 15 | 33.6 |
| 1.00 | 0.3 | 11 | 23.7 |
| Control | 0.4 | 15 | 29.0 |

(viii) Foliar application to soya bean (Glycine max)

Soya beans (variety 'Harcor') were sown one seed per peat pot in a glasshouse. Plants were selected for evenness at the first leaf stage and were planted directly one per pot (25.4 cm size) in a 'John Innes' loam-based potting compost (No. 1)/'Levington' peat-based potting compost mixture (ratio of 1:1 v/v). Plants were then grown in a polythene tunnel under a 14 hour day length regime.

At the commencement of flowering, groups of 15 plants were sprayed with solutions of various concentrations of solutions of compound A in aqueous acetone (ratio of acetone to water 4:1 v/v) to give dose rates of 0, 0.25, 0.50 and 1.00 kg/ha at a volume rate of 268 l/ha.

On maturity each plant was assessed for number of laterals and pods. Pods were oven dried at 60°C for three days and were then threshed using a laboratory thresher. The collected seeds were weighed (dry weight) and counted. 1000 seed weight, weight per pod and number of seeds per pod were calculated and the results for the mean of each group is given below in Table VIII.

TABLE VIII

| Dose (kg/ha) | Number of laterals | Number of pods | Dry weight of seed | Number of seeds |
|---|---|---|---|---|
| 0.25 | 6.4 | 67 | 17.1 | 148 |
| 0.50 | 6.5 | 61 | 13.5 | 130 |
| 1.00 | 6.1 | 57 | 10.9 | 111 |
| Control | 6.1 | 65 | 15.1 | 136 |

Cont/d.....

TABLE VIII (Continued)

| Dose (kg/ha) | Weight per pod | Seeds per pod | 1000 seed dry wt (g) |
|---|---|---|---|
| 0.25 | 0.26 | 2.2 | 116.6 |
| 0.50 | 0.23 | 2.2 | 104.2 |
| 1.00 | 0.19 | 2.0 | 98.0 |
| Control | 0.23 | 2.1 | 111.2 |

(ix) Application to winter wheat under field conditions

Winter wheat (variety 'Avalon') was drilled in October using standard farm equipment to give a row width of 17 cm and a seed rate of 186 kg/ha. NPK fertiliser (5:24:24) was applied at a rate of 250 kg/ha at drilling followed by top dressings in March and April of 250 kg/ha and 312 kg/ha respectively of Nitroshell fertilizer (34.5 units N). Herbicide and fungicide applications were made as considered necessary, to ensure a clean crop.

The trial area was laid out in 4.5 m × 2.5 m plots, with 6 replicate plots per treatment arranged randomly in blocks. Application of compound A was made using a motorised small plot crop sprayer at a volume rate of 255 l/ha, in the form of dilutions of the 25% v/v flowable formulation prepared as hereinafter described in Composition Example 7, at dose rates of 0, 0.125, 0.25 and 0.5 kg/ha.

On maturity of the crop all the stems from within three rectangular areas (each of 0.085 $m^2$) placed down the axis of each plot were removed. Each rectangular area contained three rows of plants. Heads were then counted and threshed. Seed was oven dried at 60°C for three days, weighed and counted. 1000 grain weight, dry weight per head and number of seeds per head were calculated and the mean results from the six replicate plots per treatment are shown below in Table IX.

TABLE IX

| Dose (kg/ha) | Number of heads | Dry weight of seeds (g) | Number of seeds |
|---|---|---|---|
| 0.125 | 216 | 402 | 10423 |
| 0.25 | 229 | 411 | 10763 |
| 0.50 | 226 | 432 | 11096 |
| Control | 219 | 395 | 10322 |

Cont/d.....

TABLE IX (Continued)

| Dose (kg/ha) | Dry Weight per head (g) | Seeds per head | 1000 grain dry wt (g) |
|---|---|---|---|
| 0.125 | 1.87 | 48.3 | 38.6 |
| 0.25 | 1.80 | 47.1 | 38.2 |
| 0.50 | 1.92 | 49.3 | 38.9 |
| Control | 1.81 | 47.3 | 38.3 |

(x) Application to oilseed rape under field conditions

Winter oilseed rape (variety 'Bienvenu') was drilled in September at a standard rate using normal farm equipment. NPK fertiliser (25:20:0) was applied at a rate of 250 kg/ha before emergence. A top dressing of 600 kg/ha Nitroshell fertilizer (34.5 units of N) was made in March. Herbicide, fungicide and insecticide applications were made as considered necessary to ensure a clean crop.

The trial area was laid out in 4.5 m $\times$ 2.5 m plots, with 6 replicate plots per treatment arranged randomly in blocks. Application of compound A was made using a motorised small plot crop sprayer at a volume rate of 255 l/ha. Compound A was applied, in the form of dilutions of the 25% w/v flowable formulation hereinafter described in Composition Example 7, at dose rates of 0, 0.125, 0.25 and 0.5 kg/ha.

On maturity of the crop all the stems from within a central area of 3 m $\times$ 1 m of each plot were counted and removed. Harvested plants were allowed to dry naturally under cover before hand threshing. Collected seed was passed through a seed cleaner and oven dried at 60°C for three days. Seed weight and 5000 seed weight were then measured and the mean results from the six replicate plots are shown below in Table X.

TABLE X

| dose (kg/ha) | Application at stem extension stage | | |
|---|---|---|---|
| | Number of stems | Dry weight of seed (g) | 5000 seed dry weight (g) |
| 0.125 | 145 | 1023 | 22.4 |
| 0.25 | 146 | 990 | 22.9 |
| 0.50 | 138 | 959 | 22.7 |
| Control | 137 | 834 | 22.9 |

Cont/d.....

TABLE X (Continued)

| dose (kg/ha) | Application at early flowering stage | | |
|---|---|---|---|
| | Number of stems | Dry weight of seed (g) | 5000 seed dry weight (g) |
| 0.125 | 141 | 985 | 22.9 |
| 0.25 | 136 | 972 | 23.1 |
| 0.50 | 138 | 1009 | 22.6 |
| Control | 142 | 924 | 22.0 |

(xi) Application to apple trees under field conditions

Application of compound A, in the form of dilutions of the 25% w/v flowable formulation hereinafter described in Composition Example 7, was made to apple trees (variety 'Golden Delicious') at full fruit set just after flowering. The formulation was applied, using a hand sprayer, so as to give total wetting of the leaves and fruit. Four replicate branches on two trees were treated with each concentration of 0, 250, 500 and 1000 ppm. The results are given below in Table XI.

TABLE XI

| Dose rate | No. of apples at spraying 11.6.86 | % left on tree 2.7.86 | % left at harvest 15.10.86 | Yield (kg) |
|---|---|---|---|---|
| 250 ppm | 2379 | 24 | 17.4 | 33.5 (+ 48%) |
| 500 ppm | 2426 | 24 | 17.8 | 36.5 (+ 65%) |
| 1000 ppm | 2851 | 18 | 14.9 | 32.7 (+ 44%) |
| Control | 3312 | 14 | 8.4 | 22.7 |

(xii) Utilisation in Abutilon theophrasti cell culture media instead of kinetin

A cell culture was prepared on Gamborg B5 medium with actively growing meristems removed from young Abutilon theophrasti seedlings.

This cell culture was used to inoculate other flasks containing media which were identical to Gamborg B5 medium except for the replacement of the whole of the kinetin component by 0, 2, 4, 6 or 8 mg/l of compound A. The growth of the cultures containing compound A began after 5 or 6 days, and after 12 days the cultures were harvested by filtration and weighed fresh. The results are shown hereinbelow in Table XII.

Other flasks containing 0.5, 1.0 or 2.0 mg/l of compound A instead of kinetin were then inoculated from the abovementioned culture which had contained 2 mg/l of compound A. Growth of the new cultures began after 2 or 3 days, and after 9 days these cultures were harvested and weighed fresh. The results are shown hereinafter in Table XIII.

TABLE XII

| Concentration of compound A (mg/l) | Fresh weight of culture (g) after 12 days |
|---|---|
| 0 | 1.2 |
| 2.0 | 7.3 |
| 4.0 | 7.9 |
| 6.0 | 7.6 |
| 8.0 | 7.8 |

TABLE XIII

| Concentration of compound A (mg/l) | Fresh weight of culture (g) after 9 days |
|---|---|
| 0.5 | 6.6 |
| 1.0 | 7.4 |
| 2.0 | 7.0 |

Compounds of the formula shown in Figure I are prepared by the application or adaptation of known methods, that is to say methods heretofore used or known in the literature.

For example, as a feature of the present invention, novel compounds of the general formula shown in Figure II wherein $R^9$ and $R^{10}$ each represents a cyano group or a carbamoyl group, but $R^9$ and $R^{10}$ do not simultaneously represent cyano groups, $R^1$ and $R^4$ being as hereinbefore defined, within the scope of the formula shown in Figure I, are prepared from compounds of the general formula shown in Figure III (wherein $R^1$ and $R^4$ are as hereinbefore defined) by hydrolysis, for example in the presence of an alkali metal hydroxide, e.g. sodium hydroxide, at temperatures between 15° and 100°C, to convert one of the cyano groups to a carbamoyl group.

According to a further feature of the invention, novel compounds of the general formula shown in Figure IV, wherein $R^1$, $R^2$ and $R^3$ are as hereinbefore defined but $R^1$ is preferably an optionally substituted phenyl group as hereinbefore defined, are prepared by the oxidation of compounds of the general formula shown in Figure V, wherein $R^1$, $R^2$ and $R^3$ are as hereinbefore defined. The oxidation can be carried out, for example, by reaction with an alkali metal hypohalite, e.g. sodium hypochlorite (preferably in an aqueous medium, at temperatures between 0° and 60°C), or with 2,3-dichloro-5,6-dicyanobenzoquinone (preferably at or near reflux temperature in a solvent such as acetonitrile), or with iodine (preferably in a solvent such as N-methyl-2-pyrrolidone and in the presence of sodium acetate, at an elevated temperature, e.g. at or near 100°C), or with N-chlorosuccinimide (preferably in the presence of an acid acceptor, for example an alkali metal bicarbonate, e.g. sodium bicarbonate, or nicotinamide, in a solvent such as dimethylformamide, at a temperature at or near room temperature).

Compounds of the formulae shown in Figures III and V may be prepared by the application or adaptation of known methods.

Interconversions of compounds of the formula shown in Figure I, by the application or adaptation of known methods, form a further feature of the present invention, for example:-

(i) compounds containing amino groups are prepared by the reduction of compounds containing nitro groups;

(ii) compounds containing carboxy groups are prepared by the hydrolysis of compounds containing alkoxycarbonyl groups;

(iii) compounds containing alkoxycarbonyl groups are prepared by the esterification of compounds containing carboxy groups or by the transesterification of compounds containing other alkoxycarbonyl groups;

(iv) compounds containing amino groups are converted into compounds containing alkanoylamino groups by acylation, or alternatively they are prepared from the corresponding alkanoylamino compounds by hydrolysis;

(v) compounds wherein $R^4$ represents a hydrogen atom are converted into compounds wherein $R^4$ represents an alkyl group by alkylation, for example by reaction with sodium hydride followed by reaction with the appropriate alkyl halide;

(vi) agriculturally acceptable salts as hereinbefore described are prepared by the reaction of an acidic or basic compound of the formula shown in Figure I with a suitable base or with a suitable acid respectively.

The following Examples illustrate the preparation of novel compounds of the formula shown in Figure I and the Reference Examples illustrate the preparation of intermediates.

EXAMPLE 1

Compounds A, B, C, D, E, F and G

A solution of 2-(4-chlorophenyl)-4,5-dicyanoimidazole (60.0 g; prepared as described hereinafter in Reference Example 1 or 3) in aqueous sodium hydroxide solution (1N; 650 ml) was stirred at 35-45°C for 20 hours. After treatment with decolourising charcoal the mixture was filtered through a filtration aid and the filtrate was acidified to pH 1 by treatment with hydrochloric acid (2N).

The resulting solid was filtered off, to give 2-(4-chlorophenyl)-5-cyanoimidazole-4-carboxamide (52.3 g), m.p. 343-344°C (with decomposition).

By proceeding in a similar manner, but replacing the 2-(4-chlorophenyl)-4,5-dicyanoimidazole used as starting material by the appropriate quantities of:

2-(3-methoxyphenyl)-4,5-dicyanoimidazole;
2(3-nitrophenyl)-4,5-dicyanoimidazole;
2-(4-methoxyphenyl)-4,5-dicyanoimidazole;
2-(4-nitrophenyl)-4,5-dicyanoimidazole;
2-(4-fluorophenyl)-4,5-dicyanoimidazole; and
2-(4-bromophenyl)-4,5-dicyanoimidazole;

respectively (all prepared as described hereinafter in Reference Example 1), there were prepared:-

2-(3-methoxyphenyl)-5-cyanoimidazole-4-carboxamide, m.p. 208-209°C;
2-(3-nitrophenyl)-5-cyanoimidazole-4-carboxamide, m.p. 348-349°C (with decomposition) (recrystallised from a mixture of dimethylformamide and methanol);
2-(4-methoxyphenyl)-5-cyanoimidazole-4-carboxamide, m.p. 295-297°C (recrystallised from ethanol);
2-(4-nitrophenyl)-5-cyanoimidazole-4-carboxamide, m.p. 352-353°C (with decomposition) (recrystallised from a mixture of dimethylformamide and methanol);
2-(4-fluorophenyl)5-cyanoimidazole-4-carboxamide, m.p. 336-337°C (recrystallised from methanol); and
2-(4-bromophenyl)-5-cyanoimidazole-4-carboxamide, m.p. 344-345°C (with decomposition) (recrystallised from aqueous dimethylformamide).

EXAMPLE 2

Compounds H and I

By proceeding in a manner similar to that described hereinbefore in Example 1, but replacing the 2-(4-chlorophenyl)-4,5-dicyanoimidazole used as starting material by the appropriate quantities of:-

2-(4-dimethylaminophenyl)-4,5-dicyanoimidazole hydrochloride; and
2-(4-diethylaminophenyl)-4,5-dicyanoimidazole hydrochloride;

respectively (both prepared as described hereinafter in Reference Example 2) and with the modification of precipitating the crude product at pH 6-7 by treatment with hydrochloric acid, followed by drying, dissolving in absolute ethanol, adding sufficient of a saturated solution of hydrogen chloride in diethyl ether, and filtering, there were prepared:-

2-(4-dimethylaminophenyl)-5-cyanoimidazole-4-carboxamide hydrochloride, m.p. 258-259°C (with decomposition); and

2-(4-diethylaminophenyl)-5-cyanoimidazole-4-carboxamide hydrochloride, m.p. 272-273°C (with decomposition).

## EXAMPLE 3

Compound A

A solution of 2-(4-chlorophenyl)-4,5-dicyanoimidazole (60.0 g; prepared as described hereinafter in Reference Example 1 or 3) in aqueous sodium hydroxide solution (1N; 650 ml) was stirred at 35-45°C for 20 hours. After treatment with decolourising charcoal the mixture was filtered through a filtration aid. The filtrate was diluted with an equal volume of water. It was brought to pH 6 by treatment with glacial acetic acid and then treated with saturated aqueous ammonia solution (70 ml) and heated to between 80°C and 90°C. The hot mixture was treated dropwise with glacial acetic acid until pH 6 was attained. The resulting solid was filtered off from the hot mixture, to give 2-(4-chlorophenyl)-5-cyanoimidazole-4-carboxamide (59.3 g), m.p. 343-344°C (with decomposition).

## EXAMPLE 4

Compounds J, K, L, M, N, O, P, Q, R, S, T, U, V, W, X and Y

By proceeding in a manner similar to that described hereinbefore in Example 1, but replacing the 2-(4-chlorophenyl)-4,5-dicyanoimidazole used as starting material by the appropriate quantities of:-

2-(4-acetamidophenyl)-4,5-dicyanoimidazole;
2-(4-carboxyphenyl)-4,5-dicyanoimidazole;
2-(3,4-dichlorophenyl)-4,5-dicyanoimidazole;
2-(4-methylphenyl)-4,5-dicyanoimidazole;
2-(2-methylphenyl)-4,5-dicyanoimidazole;
2-(2-trifluoromethylphenyl)-4,5-dicyanoimidazole;
2-(2-chlorophenyl)-4,5-dicyanoimidazole;
2-(4-trifluoromethylphenyl)-4,5-dicyanoimidazole;
2-(4-phenoxyphenyl)-4,5-dicyanoimidazole;
2-(3-bromophenyl)-4,5-dicyanoimidazole;
2-(3-fluorophenyl)-4,5-dicyanoimidazole;
2-(3-chlorophenyl)-4,5-dicyanoimidazole;
2-(2-fluorophenyl)-4,5-dicyanoimidazole;
2-(2,4-dichlorophenyl)-4,5-dicyanoimidazole; and
2-(4-benzyloxyphenyl)-4,5-dicyanoimidazole (all prepared as described hereinafter in Reference Example 5); and
2-(4-trifluoromethoxyphenyl)-4,5-dicyanoimidazole (prepared as described hereinafter in Reference Example 3);

respectively, there were prepared:-

2-(4-acetamidophenyl)-5-cyanoimidazole-4-carboxamide, m.p. 326-327°C (recrystallised from aqueous methanol);
2-(4-carboxyphenyl)-5-cyanoimidazole-4-carboxamide, m.p. 325-326°C;
2-(3,4-dichlorophenyl)-5-cyanoimidazole-4-carboxamide, m.p. 356-357°C (recrystallised from aqueous dimethylformamide);
2-(4-methylphenyl)-5-cyanoimidazole-4-carboxamide, m.p. 315-316°C (recrystallised from methanol);
2-(2-methylphenyl)-5-cyanoimidazole-4-carboxamide, m.p. 226-227°C (recrystallised from ethanol);
2-(2-trifluoromethylphenyl)-5-cyanoimidazole-4-carboxamide, m.p. 191-192°C;
2-(2-chlorophenyl)-5-cyanoimidazole-4-carboxamide, m.p. 209-210°C (recrystallised from aqueous ethanol);
2-(4-trifluoromethylphenyl)-5-cyanoimidazole-4-carboxamide, m.p. 326-327°C (recrystallised from methanol);
2-(4-phenoxyphenyl)-5-cyanoimidazole-4-carboxamide, m.p. 246-247°C (recrystallised from methanol);

2-(3-bromophenyl)-5-cyanoimidazole-4-carboxamide, m.p. 328-330°C (recrystallised from methanol);
2-(3-fluorophenyl)-5-cyanoimidazole-4-carboxamide, m.p. 310-311°C (recrystallised from aqueous dimethylformamide);
2-(3-chlorophenyl)-5-cyanoimidazole-4-carboxamide, m.p. 324-325°C (recrystallised from aqueous dimethylformamide);
2-(2-fluorophenyl)-5-cyanoimidazole-4-carboxamide, m.p. 261-262°C (recrystallised from ethanol;
2-(2,4-dichlorophenyl)-5-cyanoimidazole-4-carboxamide, m.p. 241-242°C (recrystallised from aqueous ethanol);
2-(4-benzyloxyphenyl)-5-cyanoimidazole-4carboxamide, m.p. 259-262°C; and
2-(4-trifluoromethoxyphenyl)-5-cyanoimidazole4-carboxamide, m.p. 290-292°C.

EXAMPLE 5

Compound Z

By proceeding in a manner similar to that described hereinbefore in Example 2, but using as the starting material the appropriate quantity of 2-(4-aminophenyl)-4,5-dicyanoimidazole (prepared as described hereinafter in Reference Example 4), there was prepared 2-(4-aminophenyl)-5-cyanoimidazole-4-carboxamide hydrochloride, m.p. above 345°C.

REFERENCE EXAMPLE 1

A mixture of cis-1-amino-2-(4-chlorobenzylideneamino)-1,2-dicyanoethylene (46.1 g), iodine (50.8 g) and anhydrous sodium acetate (33 g) in dry N-methyl-2-pyrrolidone (400 ml) was stirred and heated at 100°C for 24 hours. The reaction mixture was poured onto ice and acidified to pH 1 by treatment with concentrated hydrochloric acid. The precipitated solid was filtered off, washed with water and then with ethanol and then was treated with aqueous ammonia solution (2M; 1.5 l) whereupon most of the solid dissolved. The mixture was treated with decolourising charcoal and filtered using a filtration aid. The filtrate was then acidified to pH 1 by treatment with concentrated hydrochloric acid and the resulting precipitate was filtered off, to give 2-(4-chlorophenyl)-4,5-dicyanoimidazole (27.0 g), m.p. 293-294°C (with decomposition).

By proceeding in a similar manner, but replacing the cis-1-amino-2-(4-chlorobenzylidineamino)-1,2-dicyanoethylene used as starting material by the appropriate quantities of:-
cis-1-amino-2-(3-methoxybenzylideneamino)-1,2-dicyanoethylene;
cis-1-amino-2-(3-nitrobenzylideneamino)-1,2-dicyanoethylene;
cis-1-amino-2-(4-methoxybenzylideneamino)-1,2-dicyanoethylene;
cis-1-amino-2-(4-nitrobenzylideneamino)-1,2-dicyanoethylene;
cis -1-amino-2-(4-fluorobenzylideneamino)-1,2-dicyanoethylene; and
cis-1-amino-2-(4-bromobenzylideneamino)-1,2-dicyanoethylene;
there were prepared
2-(3-methoxyphenyl)-4,5-dicyanoimidazole, m.p. 184-185°C;
2-(3-nitrophenyl)-4,5-dicyanoimidazole, m.p. 135-136°C (recrystallised from a mixture of acetonitrile and diethyl ether);
2-(4-methoxyphenyl)-4,5-dicyanoimidazole, m.p. 231-232°C (recrystallised from ethanol);
2-(4-nitrophenyl)-4,5-dicyanoimidazole, m.p. 252-253°C;
2-(4-fluorophenyl)-4,5-dicyanoimidazole, m.p. 211-212°C (recrystallised from aqueous ethanol); and
2-(4-bromophenyl)-4,5-dicyanoimidazole, m.p. 320-321°C.

REFERENCE EXAMPLE 2

By proceeding in a manner similar to that described hereinbefore in Reference Example 1 but replacing the cis-1-amino-2-(4-chlorobenzylideneamino)-1,2-dicyanoethylene, used as starting material, by the appropriate quantities of:-
cis -1-amino-2-(4-dimethylaminobenzylideneamino)-1,2-dicyanoethylene; and
cis-1-amino-2-(4-diethylaminobenzylideneamino)-1,2-dicyanoethylene;

respectively and with the modification of precipitating the crude product at pH 4-5 by treatment with hydrochloric acid, drying, dissolving in absolute ethanol, adding sufficient of a saturated solution of hydrogen chloride in diethyl ether, and filtering, there were prepared:-

2-(4-dimethylaminophenyl)-4,5-dicyanoimidazole hydrochloride, m.p. 286-288°C (with decomposition); and 2-(4-diethylaminophenyl)-4,5-dicyanoimidazole hydrochloride, m.p. 252-254°C (with decomposition).

REFERENCE EXAMPLE 3

A solution of cis -1-amino-2-(4-chlorobenzylideneamino)-1,2-dicyanoethylene (118 g) in dimethylformamide (1 litre) at 40°C was treated with N-chlorosuccinimide (68 g). The resulting solution was then treated with sodium bicarbonate (86 g), portionwise with stirring. Evolution of carbon dioxide occurred. The mixture was stirred at 40-45°C for 20 hours and then poured into a mixture of water (2500 ml) and saturated aqueous ammonia solution (50 ml).

The resulting solution was treated with charcoal and filtered using a filtration aid. The filtrate was acidified to pH 4 by treatment with glacial acetic acid, to give 2-(4-chlorophenyl)-4,5-dicyanoimidazole (101.6 g), m.p. 295-300°C (with decomposition).

By proceeding in a similar manner, but replacing the cis-1-amino-2-(4-chlorobenzylideneamino)-1,2-dicyanoethylene used as starting material by the appropriate quantity of 1-amino-(4-trifluoromethoxybenzylideneamino)-1,2-dicyanoethylene, there was prepared 2-(4-trifluoromethoxyphenyl)-4,5-dicyanoimidazole, m.p. 182-183°C (with decomposition).

REFERENCE EXAMPLE 4

A stirred mixture of 2-(4-acetamidophenyl)-4,5-dicyanoimidazole (2.0 g) and hydrochloric acid (2N; 40 ml) was heated at reflux for 5 hours until a clear solution was formed. The mixture was cooled and filtered, and adjusted to between pH 5 and pH 6 by treatment with aqueous sodium carbonate solution (2N), to give 2-(4-aminophenyl)-4,5-dicyanoimidazole (1.0 g), m.p. 249-250°C.

REFERENCE EXAMPLE 5

By proceeding in a manner similar to that described hereinbefore in Reference Example 1 but replacing the cis-1-amino-2-(4-chlorobenzylideneamino)-1,2-dicyanoethylene, used as starting material, by the appropriate quantities of:-

cis -1-amino-2-(4-acetamidobenzylideneamino)-1,2-dicyanoethylene;
cis-1-amino-2-(4-carboxybenzylideneamino)-1,2-dicyanoethylene;
cis-1-amino-2-(3,4-dichlorobenzylideneamino)-1,2-dicyanoethylene;
cis-1-amino-2-(4-methylbenzylideneamino)-1,2-dicyanoethylene;
cis-1-amino-2-(2-methylbenzylideneamino)-1,2-dicyanoethylene;
cis-1-amino-2-(2-trifluoromethylbenzylideneamino)-1,2-dicyanoethylene;
cis-1-amino-2-(2-chlorobenzylideneamino)-1,2-dicyanoethylene;
cis -1-amino-2-(4-trifluoromethylbenzylideneamino)-1,2-dicyanoethylene;
cis-1-amino-2-(4-phenoxybenzylideneamino)-1,2-dicyanoethylene;
cis-1-amino-2-(3-bromobenzylideneamino)-1,2-dicyanoethylene;
cis-1-amino-2-(3-fluorobenzylideneamino)-1,2-dicyanoethylene;
cis -1-amino-2-(3-chlorobenzylideneamino-)-1,2-dicyanoethylene;
cis-1-amino-2-(2-fluorobenzylideneamino-)-1,2-dicyanoethylene;
cis-1-amino-2-(2,4-dichlorobenzylideneamino)-1,2-dicyanoethylene; and
cis-1-amino-2-(4-benzyloxybenzylideneamino)-1,2-dicyanoethylene;

respectively, there were prepared:-

2-(4-acetamidophenyl)-4,5-dicyanoimidazole, m.p. above 350°C;
2-(4-carboxyphenyl)-4,5-dicyanoimidazole, m.p. 307°C (recrystallised from ethanol);
2-(3,4-dichlorophenyl)-4,5-dicyanoimidazole, m.p. 294-296°C (recrystallised from acetonitrile);
2-(4-methylphenyl)-4,5-dicyanoimidazole, m.p. 257-258°C (recrystallised from aqueous ethanol);
2-(2-methylphenyl)-4,5-dicyanoimidazole, m.p. 149-150°C (recrystallised from aqueous ethanol);

2-(2-trifluoromethylphenyl)-4,5-dicyanoimidazole, m.p. 70°C;
2-(2-chlorophenyl)-4,5-dicyanoimidazole, m.p. 207-208 C (recrystallised from toluene);
2-(4-trifluoromethylphenyl)-4,5-dicyanoimidazole, m.p: 248-249°C (recrystallised from toluene);
2-(4-phenoxyphenyl)-4,5-dicyanoimidazole, m.p. 161-162°C;
2-(3-bromophenyl)-4,5-dicyanoimidazole, m.p. 225-226°C;
2-(3-fluorophenyl)-4,5-dicyanoimidazole, m.p. 185-188°C;
2-(3-chlorophenyl)-4,5-dicyanoimidazole, m.p. 219-220°C;
2-(2-fluorophenyl)-4,5-dicyanoimidazole, m.p. 244-246°C (recrystallised from aqueous ethanol);
2-(2,4-dichlorophenyl)-4,5-dicyanoimidazole, m.p. 181-182°C (recrystallised from methanol); and
2-(4-benzyloxyphenyl)-4,5-dicyanoimidazole, m.p. 257-260°C.

The dose of active ingredient applied can vary according to different factors such as, for example, the type of plant or crop to be treated, its stage of development, the effect desired, the climatic conditions and the nature of the land.

Dose rates of 0.01-10, preferably 0.5-4.0, ppm are particularly useful in plant cell tissue culture and micropropagation, 0.1-100, preferably 1.0-10, ppm as seed dressings and 10-4000, preferably 50-500, grams per hectare for application to aerial parts of the plant.

According to a feature of the present invention, there are provided compositions suitable for regulating plant growth comprising one or more of the imidazole derivatives of the general formula depicted in Figure I in association with, and preferably homogeneously dispersed in, one or more agriculturally acceptable diluents or carriers (i.e. diluents or carriers of the type generally accepted in the art as being suitable for use in compositions and which are compatible with compounds of the general formula depicted in Figure I). The term "homogeneously dispersed" is used to include compositions in which the compounds of the general formula depicted in Figure I are dissolved in the other components. The term "compositions" is used in a broad sense to include not only compositions which are ready for use but also concentrates which must be diluted before use. Preferably, the compositions contain from 0.05 to 90% by weight of one or more compounds of the general formula depicted in Figure I.

The compositions may contain both a diluent or carrier and one or more surface-active (e.g. wetting, dispersing, or emulsifying) agents. Surface-active agents which may be present in compositions of the present invention may be of the ionic or non-ionic types, for example sulphoricinoleates, quaternary ammonium derivatives, products based on condensates of ethylene oxide with alkyl and polyaryl phenols, e.g. nonyl-or octyl-phenols, or carboxylic acid esters of anhydrosorbitols whose solubility has been modified by etherification of the free hydroxy groups by condensation with ethylene oxide, alkali and alkaline earth metal salts of sulphuric acid esters and sulphonic acids such as sodium dinonyl-and dioctyl-sulphonosuccinates and alkali and alkaline earth metal salts of high molecular weight sulphonic acid derivatives such as sodium and calcium lignosulphonates and sodium and calcium alkylbenzenesulphonates.

Compositions which comprise a compound of the general formula shown in Figure I preferably comprise a surface-active agent.

Suitably, the compositions according to the present invention may comprise up to 10%, e.g. from 0.05% to 10%, of surface-active agent but, if desired, compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of the general formula depicted in Figure I with solid diluents or by impregnating the solid diluents or carriers with solutions of the compounds of the general formula depicted in Figure I in volatile solvents, evaporating the solvents and, if necessary, grinding the products so as to obtain powders. Granular formulations may be prepared by absorbing the compounds of the general formula depicted in Figure I (dissolved in suitable solvents, which may, if desired, be volatile) onto the solid diluents or carriers in granular form and, if desired, evaporating the solvents, or by granulating compositions in powder form obtained as described above. Solid compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents,

which may be present in the liquid compositions, may be ionic or non-ionic (for example of the types described above) and may, when liquid, also serve as diluents or carriers.

In liquid compositions which comprise a known compound of the general formula shown in Figure I the liquid diluent is preferably other than water or a common organic solvent.

Wettable powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use. When desired, liquid compositions of the compounds of the general formula depicted in Figure I may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred compositions according to the present invention are aqueous suspension concentrates which comprise from 10 to 70% of one or more compounds of the general formula depicted in Figure I, from 2 to 10% of surface-active agent, from 0.1 to 5% w/v of thickener and from 15 to 87.9% by volume of water; wettable powders which comprise from 5 to 88% w/w of one or more compounds of the general formula depicted in Figure I, from 2 to 10% w/w of surface-active agent and from 10 to 93% w/w of solid diluent or carrier; liquid water soluble concentrates which comprise from 1 to 50%, e.g. 1 to 15%, w/v of one or more compounds of the general formula depicted in Figure I, from 5 to 25% w/v of surface-active agent and from 25 to 90%, e.g. 45 to 85%, by volume of water-miscible solvent, e.g. N-methylpyrrolidone or dimethylformamide, or a mixture of water-miscible solvent and water; liquid emulsifiable suspension concentrates which comprise from 5 to 70% w/v of one or more compounds of the general formula depicted in Figure I, 5 to 15% w/v of surface active agent, from 0.1 to 5% w/v of thickener and from 10 to 89.9% by volume of organic solvent; granules which comprise from 1 to 90%, e.g. 2 to 10%, w/w of one or more compounds of the general formula depicted in Figure I, from 0.5 to 7%, e.g. 0.5 to 2%, w/w of surface-active agent and from 3 to 98.5%, e.g. 88 to 97.5%, w/w of granular carrier and emulsifiable concentrates which comprise 0.05 to 90% w/v, and preferably from 1 to 15% of one or more compounds of the general formula depicted in Figure I, from 0.01 to 10% w/v, and preferably from 1 to 10% w/v, of surface-active agent and from 9.99 to 99.94%, and preferably from 75 to 98%, by volume of organic solvent.

Compositions according to the present invention may also comprise the compounds of the general formula depicted in Figure I in association with, and preferably homogenously dispersed in, one or more pesticidally active compounds and, if desired, one or more compatible pesticidally acceptable diluents or carriers, surface-active agents and conventional adjuvants as hereinbefore described. Examples of pesticidally active compounds which may be included in, or used in conjunction with, the compositions of the present invention, include insecticides, e.g. carbaryl [naphth-1-yl-N-methylcarbamate]; synthetic pyrethroids, e.g. permethrin and cypermethrin; and fungicides, e.g. 2,6-dimethyl-4-tridecylmorpholine, methyl N-(1-butylcarbamoyl-benzimidazol-2-yl)carbamate, 1,2-bis-(3-methoxycarbonyl-2-thioureido)benzene, isopropyl-1-carbamoyl-3-(3,5-dichlorophenyl)hydantoin and 1-(4-chloro-phenoxy)-3,3-dimethyl-1-(1,2,4-triazol-1-yl)butan-2-one. Other biologically active materials which may be included in, or used in conjunction with, the compositions of the present invention are other plant growth regulators, e.g. succinamic acid, (2-chloroethyl)trimethylammonium chloride and 2-chloroethane-phosphonic acid; or fertilizers and seaweed extracts, e.g. containing nitrogen, potassium and phosphorus and trace elements known to be essential to successful plant life, e.g. iron, magnesium, zinc, manganese, cobalt and copper.

Pesticidally active compounds and other biologically active materials which may be included in, or used in conjunction with, the compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the compounds of the general formula depicted in Figure I or, as is preferred, a composition as herein described, and preferably a concentrate which must be diluted before use, comprising at least one of the compounds of the general formula depicted in Figure I within a container for the aforesaid derivative or derivatives of the general formula depicted in Figure I, or a said composition, and instructions physically associated with the aforesaid container setting out the manner in which the aforesaid

derivative or derivatives of the general formula depicted is Figure I or composition contained therein is to be used to regulate the growth of plants. The containers will normally be of the types conventionally used for the storage of chemical substances which are solid at normal ambient temperatures and compositions particularly in the form of concentrates, for example cans and drums of metal, which may be internally-lacquered, and plastics materials, bottles of glass and plastic materials and, when the contents of the container is a solid, for example granular plant growth regulator compositions, boxes, for example of cardboard, plastics materials and metal, or sacks. The containers will normally be of sufficient capacity to contain amounts of the imidazole derivative or compositions sufficient to treat at least one acre of ground to regulate the growth of plants therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to regulate the growth of plants, for example, at the rates of application and for the purposes hereinbefore described.

According to a further feature of the invention there are provided seeds having a dressing comprising a compound of the general formula shown in Figure I. The dressing may be applied by known methods, for example as hereinbefore described in Test (v).

The invention also provides a plant-cell culture medium comprising a compound of the general formula shown in Figure I.

The following Composition Examples illustrate compositions according to the present invention. The compositions described in Composition Examples 1 to 7 can each be diluted in water to give a sprayable composition at concentrations suitable for use in the field.

COMPOSITION EXAMPLE 1

A water soluble concentrate was prepared from
2-(4-chlorophenyl)-5-cyanoimidazole-4-carboxamide 7% w/v
Ethylan BCP 10% w/v
and N-methylpyrrolidone to 100% by volume
by dissolving the Ethylan BCP in a portion of N-methylpyrrolidone, and then adding the active ingredient with heating and stirring until dissolved. The resulting solution was made up to volume by adding the remainder of the solvent.

COMPOSITION EXAMPLE 2

An emulsifiable concentrate was prepared from
2-(4-chlorophenyl)-5-cyanoimidazole-4-carboxamide 7% w/v
Soprophor BSU 4% w/v
Arylan CA 4% w/v
N-methylpyrrolidone 50% w/v
and Solvesso 150 to 100% by volume
by dissolving Soprophor BSU, Arylan CA and the active ingredient in N-methylpyrrolidone, and then adding Solvesso 150 to volume.

COMPOSITION EXAMPLE 3

A wettable powder was prepared from
2-(4-chlorophenyl)-5-cyanoimidazole-4-carboxamide 40% w/v
Arylan S 2% w/v
Darvan No. 2 5% w/v
and Celite PF to 100% by weight
by mixing the ingredients, and grinding the mixture in a hammer-mill to a particle size less than 50 microns.

COMPOSITION EXAMPLE 4

An aqueous flowable formulation was prepared from
2-(4-chlorophenyl)-5-cyanoimidazole-4-carboxamide 30% w/v
Ethylan BCP 1% w/v
Sopropon T36 0.2% w/v
Ethylene glycol 5% w/v
Rhodigel 23 0.15% w/v
and water to100% by volume
by intimately mixing the ingredients and grinding in a bead mill until the median particle size was less than 3 microns.

COMPOSITION EXAMPLE 5

An emulsifiable suspension concentrate was prepared from
2-(4-chlorophenyl)-5-cyanoimidazole-4-carboxamide 30% w/v
Ethylan BCP 10% w/v
Bentone 38 0.5% w/v
and Solvesso 150 to 100% by volume
by intimately mixing the ingredients and grinding in a bead mill until the median particle size was less than 3 microns.

COMPOSITION EXAMPLE 6

Water dispersible granules were prepared from
2-(4-chlorophenyl)-5-cyanoimidazole-4-carboxamide 30% w/v
Darvan No. 2 15% w/v
Arylan S 8% w/v
and Celite PF to 100% by weight
by mixing the ingredients, micronising in a fluid-energy mill, and then granulating in a rotating pelletiser by spraying on sufficient water (up to 10% w/w). The resulting granules were dried in a fluid-bed drier to remove excess water.

COMPOSITION EXAMPLE 7

An aqueous flowable formulation was prepared from
2-(4-chlorophenyl)-5-cyanoimidazole-4-carboxamide 25% w/v
propylene glycol 5% w/v
Soprophor FL 1.5% w/v
Vanisperse CB 1.5% w/v
Attagel 50 1% w/v
Ethylan BCP 0.5% w/v
Sopropon T36 0.4% w/v
anhydrous sodium carbonate 0.025% w/v
Antifoam FD 0.1% w/v
and water to100%
by dissolving the Soprophor FL and the Ethylan BCP in the propylene glyccl, adding the Sopropon T36, the Vanisperse CB and the Attagel 50 with stirring, followed by the Antifoam FD and some water (70 volumes). The 2-(4-chlorophenyl)-5-cyanoimidazole-4-carboxamide was slowly added, followed by the anhydrous sodium carbonate. The bubbles were allowed to escape and the mixture was made up to volume by the addition of the rest of the water, and ground in a bead-mill until the particle size was less than 10.5 microns.

Descriptions of commercial ingredients used in the foregoing Composition Examples:-Antifoam FD a silicone antifoam

Arylan CA 70% w/v solution of calcium dodecylbenzenesulphonate
Arylan S sodium dodecylbenzenesulphonate
Attagel 50 attapulgite clay
Bentone 38 organic derivative of magnesium montmorillonite
Celite PF synthetic magnesium silicate carrier
Darvan No. 2 sodium lignosulphonate
Ethylan BCP nonylphenol ethylene oxide condensate
Rhodigel 23 polysaccharide xanthan gum
Solvesso 150 light $C_{10}$-aromatic solvent
Soprophor BSU condensate of tristyrylphenol and ethylene oxide
Soprophor FL triethanolamine salt of an oxyethylated polyarylphenol phosphate
Sopropon T36 sodium salt of polycarboxylic acid
Vanisperse CB sodium lignosulphonate

Similar compositions may be prepared by replacing the Compound A in the Composition Examples by the appropriate quantity of any of the compounds B to Z.

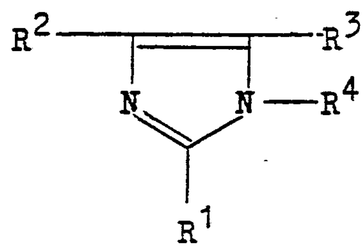

FIG. I

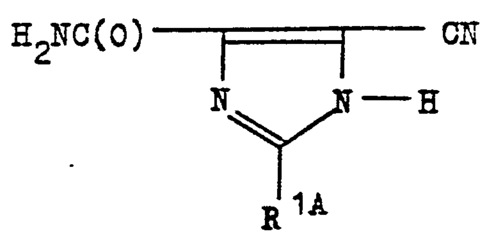

FIG. IA

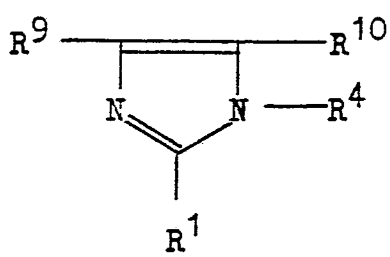

FIG. II

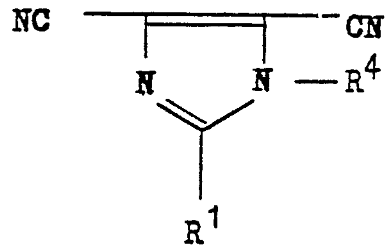

FIG. III

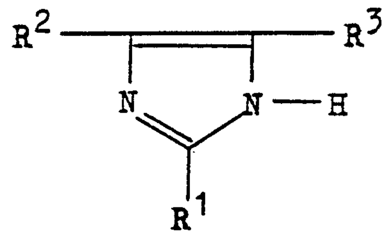

FIG. IV

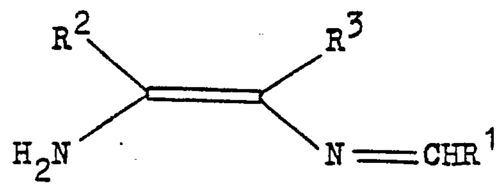

FIG. V

## Claims

A method of regulating the growth of a plant or of plant material at a locus which comprises applying to the plant, to plant material or to the locus an amount, effective to regulate growth, of an imidazole derivative of the general formula:

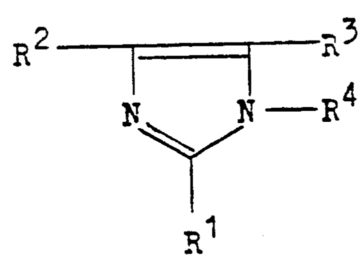

I

wherein $R^1$ represents a straight-or branched-chain alkyl group containing from 1 to 8 carbon atoms or a phenyl group which is optionally substituted by one or more substituents selected from halogen atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, alkoxycarbonyl, alkylamino and dialkylamino groups in which the alkyl moieties are each straight or branched and contain from 1 to 6 carbon atoms, and straight-or branched-chain alkanoylamino groups containing from 2 to 5 carbon atoms, $R^2$ represents a nitro, cyano or carbamoyl group or a group of the formula $-CONR^5R^6$ or $-NR^6COR^5$(wherein $R^5$ is as hereinbefore defined for $R^1$, and $R^6$ represents a hydrogen atom or a straight-or branched-chain alkyl group containing from 1 to 6 carbon atoms), $R^3$ represents a

halogen atom or a nitro, cyano, amino or carbamoyl group or a group of the formula $-CONR^7R^8$ (wherein $R^7$ is as hereinbefore defined for $R^1$ and $R^8$ represents a hydrogen atom or a straight-or branched-chain alkyl group containing from 1 to 6 carbon atoms), and $R^4$ represents a hydrogen atom or a straight-or branched-chain alkyl group containing from 1 to 6 carbon atoms, or an agriculturally acceptable salt thereof, with the exclusion of compounds in which $R^2$ and $R^3$ simultaneously represent cyano groups.

2. A method according to claim 1 which comprises:

(a) to increase the yield of a root, seed or fruit crop at a locus applying thereto an effective amount of a compound of general formula I or a salt thereof;

(b) dressing seed, prior to planting, with a composition comprising a compound of general formula I or a salt thereof; or

(c) to increase the growth of a plant cell culture growing the cell culture in a medium comprising a compound of general formula I or a salt thereof.

3. A method according to claim 1 or 2 in which, in the compound of the general formula I, $R^1$ represents a phenyl group optionally bearing one or two substituents selected from halogen atoms, trifluoromethyl, trifluoromethoxy, nitro, amino, carboxy, phenoxy and benzyloxy groups, and alkyl, alkoxy and dialkylamino groups in which the alkyl moieties are straight-or branched-chain and each contains from 1 to 6 carbon atoms, and straight-or branched-chain alkanoylamino groups containing from 2 to 5 carbon atoms, $R^2$ represents a carbamoyl or cyano group and $R^3$ represents a carbamoyl or cyano group, and $R^4$ represents a hydrogen atom.

4. A method according to claim 1, 2 or 3 in which the compound of general formula I is 2-(4-chlorophenyl)-5-cyanoimidazole-4-carboxamide.

5. A composition suitable for plant growth regulation which comprises an imidazole derivative of general formula I as defined in claim 1 or an agriculturally acceptable salt thereof in association with an agriculturally acceptable diluent or carrier.

6. A composition according to claim 5which is a seed dressing or a plant-cell culture medium.

7. A composition according to claim 5 or 6 in which the compound of general formula I is as defined in claim 1 or an agriculturally acceptable salt thereof with the exclusion of:

compounds of the general formula shown in Figure I wherein $R^1$ represents an unsubstituted phenyl group, $R^2$ represents a cyano group, $R^3$ represents a carbamoyl group and $R^4$ represents a hydrogen atom;

compounds of the general formula shown in Figure I wherein $R^1$ represents a tert-butyl or isopropyl group, or a 4-methylphenyl, 4-chlorophenyl, 4-acetamidophenyl or 4-methoxyphenyl group, $R^2$ and $R^3$ may be the same or different and each represents a cyano or carbamoyl group or a group of the formula $-CONR^5R^6$ or $-CONR^7R^8$ (wherein $R^5$ or $R^7$ represents an alkyl group and $R^6$ or $R^8$, represents a hydrogen atom), and $R^4$ represents a hydrogen atom, and salts thereof;

compounds of the general formula shown in Figure I wherein $R^1$ represents an alkyl group containing from 1 to 4 carbon atoms, $R^2$ represents a nitro or cyano group, $R^3$ represents a halogen atom or a nitro, cyano or amino group and $R^4$ represents a hydrogen atom or an alkyl group containing from 1 to 4 carbon atoms;

compounds of the general formula shown in Figure I wherein $R^1$ represents an alkyl group containing from 1 to 4 carbon atoms, $R^2$ represents a nitro or cyano group, $R^3$ represents a halogen atom or a nitro or cyano group and $R^4$ represents a hydrogen atom or an alkyl group;

compounds of the general formula shown in Figure I wherein $R^1$ represents an alkyl group, $R^2$ and $R^3$ each represent carbamoyl groups and $R^4$ represents a hydrogen atom;

compounds of the general formula shown in Figure I wherein $R^1$ represents an alkyl group, $R^2$ represents a group of the formula $-NR^6COR^5$ (wherein $R^5$ represents an alkyl group containing from 1 to 7 carbon atoms and $R^6$ represents a hydrogen atom), $R^3$ represents a carbamoyl group or a group of the formula $-CONR^7R^8$ (wherein $R^7$ represents an alkyl group containing from 1 to 4 carbon atoms and $R^8$ represents a hydrogen atom or an alkyl group containing from 1 to 4 carbon atoms) and $R^4$ represents a hydrogen atom or an alkyl group;

compounds of the general formula shown in Figure I wherein $R^1$ represents an alkyl group, $R^2$ represents a carbamoyl group or a group of the formula $-CONR^5R^6$ (wherein $R^5$ represents an alkyl group containing from 1 to 4 carbon atoms and $R^6$ represents a hydrogen atom or an alkyl group containing from 1 to 4 carbon atoms), $R^3$ represents an amino group and $R^4$ represents a hydrogen atom or an alkyl group;

compounds of the general formula shown in Figure I wherein $R^1$ represents a phenyl group optionally substituted on its 3-, 4-, 5-and 6-positions by one or two substituents selected from halogen atoms, alkyl groups containing from 1 to 4 carbon atoms and alkoxy, trifluoromethyl, nitro, amino, carboxy, phenoxy, benzyloxy, alkoxycarbonyl, alkylamino, dialkylamino and alkanoylamino groups, $R^2$ represents a group of the formula $-CONR^5R^6$ [wherein $R^5$ represents a straight-or branched-chain alkyl group containing from 1 to

8 carbon atoms or a phenyl group which is optionally substituted by one or more substituents selected from halogen atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, and alkoxycarbonyl, alkylamino and dialkylamino groups in which the alkyl moieties are each straight or branched and contain from 1 to 6 carbon atoms, and straight-or branched-chain alkanoylamino groups containing from 2 to 5 carbon atoms, and $R^6$ represents a hydrogen atom or a straight- or branched-chain alkyl group containing from 1 to 6 carbon atoms], $R^3$ represents an amino group and $R^4$ represents a hydrogen atom, and salts thereof;

compounds of the general formula shown in Figure I wherein $R^1$ represents a phenyl group optionally substituted by a halogen atom or a methyl, methoxy, trifluoromethyl or nitro group, $R^2$ represents a group of the formula $-NR^6COR^5$ (wherein $R^5$ represents an alkyl group containing 1 to 7 carbon atoms and $R^6$ represents a hydrogen atom), $R^3$ represents a carbamoyl group and $R^4$ represents a hydrogen atom;

compounds of the general formula shown in Figure I wherein $R^1$ represents a phenyl group optionally substituted on its 3-, 4-, 5-and 6-positions by one or two substituents selected from halogen atoms, alkyl groups containing from 1 to 4 carbon atoms and alkoxy, trifluoromethyl, nitro, amino, carboxy, phenoxy, benzyloxy, alkoxycarbonyl, alkylamino, dialkylamino and alkanoylamino groups, $R^2$ represents a group of the formula $-NR^6COR^5$ (wherein $R^5$ represents an alkyl group containing from 1 to 7 carbon atoms and $R^6$ represents a hydrogen atom or an alkyl group), $R^3$ represents a group of the formula $-CONR^7R^8$ [wherein $R^7$ represents a straight-or branched-chain alkyl group containing from 1 to 8 carbon atoms or a phenyl group which is optionally substituted by one or more substituents selected from halogen atoms, straight-or branched-chain alkyl, alkoxy and alkylthio groups containing from 1 to 6 carbon atoms, trifluoromethyl, trifluoromethoxy, trifluoromethylthio, nitro, cyano, amino, carboxy, phenoxy and benzyloxy groups, and alkoxycarbonyl, alkylamino and dialkylamino groups in which the alkyl moieties are each straight or branched and contain from 1 to 6 carbon atoms, and straight-or branched-chain alkanoylamino groups containing from 2 to 5 carbon atoms, and $R^8$ represents a hydrogen atom or a straight-or branched-chain alkyl group containing from 1 to 6 carbon atoms], and $R^4$ represents a hydrogen atom, and salts thereof; and

compounds of the formula shown in Figure I wherein $R^1$ represents an alkyl group containing from 1 to 5 carbon atoms or a phenyl group substituted by one or more substituents selected from halogen atoms and nitro groups, $R^2$ represents a nitro group, $R^3$ represents a cyano group, and $R^4$ represents an alkyl group containing from 1 to 5 carbon atoms.

8. A composition according to claim 5, 6 or 7 in which the compound of general formula I is a compound of the general formula:

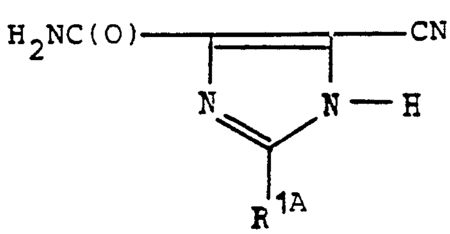

IA

wherein $R^{1A}$ represents a phenyl group substituted by one or two substituents selected from those listed in the definition of $R^1$ in claim 1.

9. A composition according to any one of claims 5, 6, 7 or 8 which is an aqueous suspension concentrate which comprises from 10 to 70% of one or more compounds of the general formula I, from 2 to 10% of surface-active agent, from 0.1 to 5% w/v of thickener and from 15 to 87.9% by volume of water;

a wettable powder which comprises from 5 to 88% w/w of one or more compounds of the general formula I, from 2 to 10% w/w of surface-active agent and from 10 to 93% w/w of solid diluent or carrier;

a liquid water soluble concentrate which comprises from 1 to 50% w/v of one or more compounds of the general formula I, from 5 to 25% w/v of surface-active agent and from 25 to 90% by volume of water-miscible solvent, or a mixture of water-miscible solvent and water;

a liquid emulsifiable suspension concentrate which comprises from 5 to 70% w/v of one or more compounds of the general formula I, 5 to 15% w/v of surface active agent, from 0.1 to 5% w/v of thickener and from 10 to 89.9% by volume of organic solvent;

granules which comprise from 1 to 90% w/w of one or more compounds of the general formula I, from 0.5 to 7% w/w of surface-active agent and from 3 to 98.5% w/w of granular carrier; or

an emulsifiable concentrate which comprises 0.05 to 90% w/v, of one or more compounds of the general formula I, from 0.01 to 10% w/v of surface-active agent and from 9.99 to 99.94% by volume of organic solvent.

10. Seeds having a dressing comprising a compound of general formula I or an agriculturally acceptable salt thereof.

11. A compound of general formula I as defined in claim 7 or a salt thereof.

12. A compound according to claim 11 in which the compound of general formula I is a compound of the general formula:

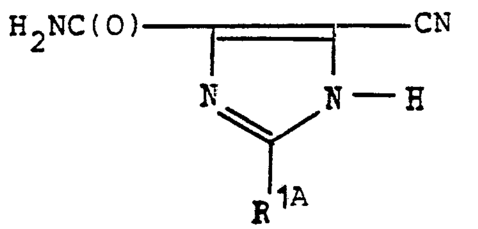

IA

wherein $R^{1A}$ represents a phenyl group substituted by one or two substituents selected from those listed in the definition of $R^1$ in claim 1.

13. A compound of formula I which is 2-(4-chlorophenyl)-5-cyanoimidazole-4-carboxamide, 2-(4-methoxyphenyl)-5-cyanoimidazole-4-carboxamide, 2-(4-acetamidophenyl)-5-cyanoimidazole-4-carboxamide or 2-(4-methylphenyl)-5-cyanoimidazole-4-carboxamide.

14. A process for the preparation of a compound according to claim 11 which comprises:

(a) when the compound is of the general formula:

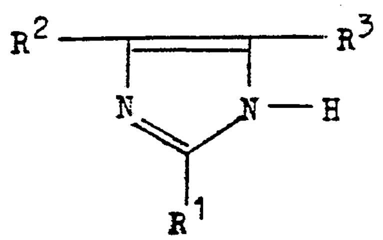

IV

wherein $R^1$, $R^2$ and $R^3$ are as defined in claim 7 which comprises the oxidation of a compound of the general formula:

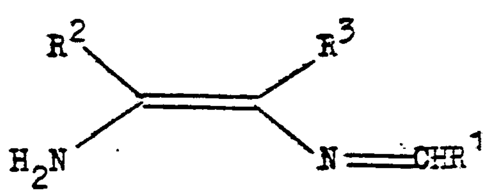

1

wherein $R^1$, $R^2$ and $R^3$ are as defined in claim 7; or

(b) when the compound is of the general formula:

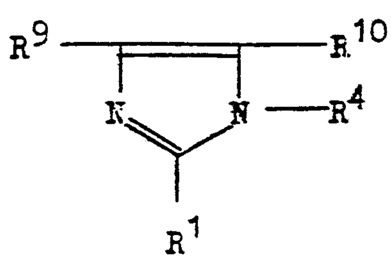

II

wherein $R^9$and $R^{10}$ each represents a cyano group or a carbamoyl group, but $R^9$ and $R^{10}$ do not simultaneously represent cyano groups, $R^1$ and $R^4$ being as defined in claim 7, the hydrolysis of a compound of the general formula:

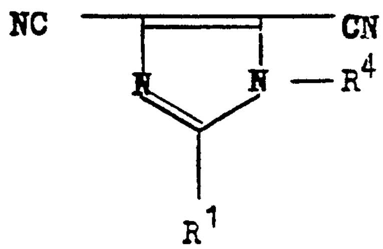

III

wherein $R^1$ and $R^4$ are as defined in claim 7, to convert one of the cyano groups to a carbamoyl group; optionally followed by:

(i) the reduction of a compound of formula I containing a nitro group to a compound of formula I containing an amino group;

(ii) the hydrolysis of a compound of formula I containing an alkoxycarbonyl group to a compound of general formula I containing a carboxy group;

(iii) the esterification or transesterification of a compound of formula I containing a carboxy group or an alkoxycarbonyl group to a compound of formula I containing an alkoxycarbonyl group;

(iv) the acylation of a compound of formula I containing an amino group to a compound of formula I containing an alkanoylamino group or the hydrolysis of a compound of formula I containing an alkanoylamino group to a compound of formula I containing an amino group;

(v) the alkylation of a compound of formula I in which $R^4$ represents a hydrogen atom to a compound of formula I in which $R^4$ represents an alkyl group and/or

(vi) the conversion of a compound of formula I into a salt thereof.

European Patent Office

# PARTIAL EUROPEAN SEARCH REPORT

which under Rule 45 of the European Patent Convention shall be considered, for the purposes of subsequent proceedings, as the European search report

Application number
EP 87 30 9141

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DD - A - 140 966 (D. CREUZBURG et al.)<br>* Claims, page 4, lines 3-29; page 18, compounds 137-145; page 19, compounds 151-153 *<br>-- | 1-6,10 | A 01 N 43/50<br>C 07 D 233/92<br>C 07 D 233/90 |
| D,A | US - A - 3 501 286 (W. DRABER et al.)<br>* Whole document *<br>-- | 1-6,10 | |
| D,A | JOURNAL OF ORGANIC CHEMISTRY, vol. 44, no. 5, 2nd March 1979, Easton, Penn. US;<br>Y. OHTSUKA: "Chemistry of diaminomaleonitrile.4.Nitrile hydration of the Schiff Bases[1]"<br>* Page 828, left-hand column, last two lines - right-hand column, first line *<br>-- | 1-6 | |
| D,A | US - A - 3 968 228 (D.R. HOFF et al.)<br>* Column 3, lines 43-59; column 4, lines 1-68 *<br>-- | 1-6 | TECHNICAL FIELDS SEARCHED (Int. Cl.4)<br>A 01 N<br>C 07 D |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6,10
Claims searched incompletely:
Claims not searched: 7-9,11-14
Reason for the limitation of the search:

Art. 83 & 84

(i) Discordance between claims 5 and 6, and claims 7-9

(ii) Absence of clarity due to the number of different disclaimers in claim 7

| Place of search | Date of completion of the search | Examiner |
|---|---|---|
| The Hague | 01-02-1988 | FLETCHER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1505.1. 03.82

European Patent Office

# PARTIAL EUROPEAN SEARCH REPORT

Application number EP 87 30 9141



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 1 390 402 (BEECHAM GROUP LTD) | | |
| | * Page 1, line 31 - page 2, line 19; examples; claims 1-13 * | 1-6 | |
| D,A | & US - A - 3 959 305 | | |
| | -- | | |
| D,A | US - A - 3 836 658 (J.J. BALDWIN et al.) | | |
| | * Column 2, lines 8-41; table 2; examples 13-17,19; claim 1 * | 1-4 | |
| | -- | | |
| A | US - A - 3 644 391 (W.A. SKLARZ et al.) | | TECHNICAL FIELDS SEARCHED (Int Cl.4) |
| | * Column 4, lines 74,75; claim 1 * | 1-4 | |
| | -- | | |
| A | GB - A - 1 290 693 (KRKA TOVARNA ZDRAVIL) | | |
| | * Examples 13,14; claims * | 1-4 | |
| D,A | & BE - A - 741 077 | | |
| | -------------------- | | |